# EUROPEAN PATENT APPLICATION

(11) **EP 4 699 631 A1**
(43) Date of publication of application: **25.02.2026**
(21) Application number: 24306378.1
(22) Date of filing: 20.08.2024
(51) Int. Cl.: A61M 5/315

(54) **STOPPER WITH IMPROVED TRIM MEMBER FEATURES AND GLIDING PERFORMANCE**

(71) Applicant: Becton Dickinson France, 38800 Le Pont-de-Claix (FR)
(72) Inventor: VOIRIN, Coralie, 38360 Sassenage (FR); FONBLANC, Diane, 38080 L'isle d'Abeau (FR); GONTARD, Lucile, 38760 Varces-Allieres-et-Risset (FR)
(74) Representative: Germain Maureau

(57) **Abstract**

A stopper (10) adapted for use within a syringe barrel, the stopper including a main body (12) defining a proximal end (14), a distal end (16), and a cylindrical sidewall (20) extending between the proximal end and the distal end, wherein the main body defines a first diameter; at least one rib (22) extending radially outward around a perimeter of the main body configured to form a seal with the syringe barrel; and a trim member (24) extending radially outward around a perimeter of the main body, the trim member including a distal portion (28) defining a second diameter, a proximal portion (26) defining a third diameter, and an intermediate portion (30) between the distal portion and the proximal portion, the intermediate portion defining a fourth diameter at least 1% smaller than the second diameter and at least 0.5% smaller than the third diameter.

## Description

### BACKGROUND OF THE INVENTION

### Field of the Invention

The present disclosure is directed to a stopper or a series of stoppers for use with injection devices, such as syringes, cartridges, and/or auto-injectors, having trim member features that improve gliding performance.

### Description of Related Art

Devices for automatic injection of a product, also called auto-injectors, are widely used in medical fields where the treatment of pathology requires daily injections, such as the treatment of some diabetes or arthritis. Auto-injectors usually comprise, on one hand, a container filled with the product to be injected and a needle, such as a prefilled syringe or a cartridge, and on the other hand, a motor part comprising the various systems which will trigger the insertion of the needle, actuate the injection, and potentially activate a protection system at the end of injection.

Syringes, either prefilled or prefillable, auto-injectors, and other types of injection devices, often require slow and controlled initiation and maintenance of sliding movement of one surface over another surface, e.g., a stopper and/or plunger moving through a barrel or container of medicament. Two stationary surfaces having a sliding relationship often exhibit sufficient resistance to the initiation of movement that gradually increased force applied to one of the surfaces does not cause movement until a threshold force is reached, at which point a sudden sliding or shearing separation of the surfaces takes place. This sudden separation of stationary surfaces into a sliding relationship is herein referred to as "breakout" or "breakloose." Often times, this breakout or breakloose is partly caused by a concentration of stress between the trim member of a stopper, (i.e., the location where the individual stoppers are trimmed from a molding sheet) that is in contact with the inner surface of the syringe barrel. "Breakout force" refers to the force required to overcome static friction between surfaces of a syringe assembly that has been previously moved in a sliding relationship, but has been stationary ("parked" or not moved) for a short period of time (for example, milliseconds to hours). Another frictional force is "breakloose force," which refers to the force required to overcome static friction between surfaces of a syringe assembly that have not been previously moved in a sliding relationship or have been stationary for longer periods of time, often with chemical or material bonding or deformation of the surfaces due to age, sterilization, temperature cycling, or other processing.

Another important feature of an injection device is that the stopper should have good gliding properties i.e., a low "gliding force" to move the stopper within the container. The gliding force of a stopper within either a filled or empty syringe is directly related to an important parameter of the syringe - the injection time of the drug contained in the syringe. This key parameter is especially important for syringes used in auto-injectors, as it must comply with a defined injection time. Some stopper designs may include high variability regarding their breakloose and gliding performances which are linked to the diameter, shape, and eccentricity of the trimming surfaces, which can result in undesirable variations and lack of uniformity in resulting injection times.

A conventional approach to overcoming breakout, breakloose, and gliding forces has been the application of a lubricant, such as silicone or hydrocarbon oils, to a surface interface. The use of a lubricant may enhance or reduce the gliding forces needed to move the stopper within the syringe barrel. However, the use of some lubricants, especially when used with high-value biotech drugs, can have deleterious effects on these drugs. Thus, there is a need in the art to consistently control and/or reduce the gliding forces required to move a stopper within an auto-injector, syringe, and the like, while consistently controlling and/or reducing the amount of lubricant needed to achieve these reduced gliding forces.

### SUMMARY OF THE INVENTION

In accordance with one aspect, the present disclosure provides a stopper adapted for use within a syringe barrel, the stopper comprising: a main body defining a proximal end, a distal end, and a cylindrical sidewall extending between the proximal end and the distal end, wherein the main body defines a first diameter; at least one rib extending radially outward around a perimeter of the main body configured to form a seal with the syringe barrel; and a trim member extending radially outward around a perimeter of the main body, the trim member comprising: a distal portion defining a second diameter, a proximal portion defining a third diameter, and an intermediate portion between the distal portion and the proximal portion, the intermediate portion defining a fourth diameter at least 1% smaller than the second diameter and at least 0.5% smaller than the third diameter.

In some non-limiting embodiments or aspects, the second diameter may be less than or equal to 9.15 mm.

In some non-limiting embodiments or aspects, the second diameter may be between 8.85 mm and 9.16 mm.

In some non-limiting embodiments or aspects, the fourth diameter may be between 8.85 mm and 9.08 mm.

In some non-limiting embodiments or aspects, the third diameter may be between 8.85 mm and 9.12 mm.

In some non-limiting embodiments or aspects, the main body may define a first diameter between 8.85 mm and 9.05 mm.

In some non-limiting embodiments or aspects, the distal portion may include a convex segment.

In some non-limiting embodiments or aspects, at least one of the proximal portion and the intermediate portion may include a concave segment.

In some non-limiting embodiments or aspects, a radius of curvature of the concave segment may be greater than a radius of curvature of the convex segment.

In some non-limiting embodiments or aspects, the second diameter may be less than the third diameter.

In some non-limiting embodiments or aspects, the trim member may include a concave segment extending from the distal portion to the proximal portion.

In some non-limiting embodiments or aspects, the second diameter may be less than the third diameter.

In accordance with one aspect, the present disclosure provides a stopper adapted for use within a syringe barrel, the stopper comprising: a main body defining a proximal end, a distal end, and a cylindrical sidewall extending between the proximal end and the distal end, wherein the main body defines a first diameter; at least one rib extending radially outward around a perimeter of the main body configured to form a seal with the syringe barrel; and a trim member extending radially outward around a perimeter of the main body, the trim member comprising: a distal portion including a convex segment, a proximal portion, and an intermediate portion between the distal portion and the proximal portion, wherein at least one of the proximal portion and the intermediate portion includes a concave segment.

In some non-limiting embodiments or aspects, a radius of curvature of the concave segment may be greater than a radius of curvature of the convex segment.

In some non-limiting embodiments or aspects, the radius of curvature of the convex segment may be between 0.3 mm and 0.7 mm.

In some non-limiting embodiments or aspects, the radius of curvature of the concave segment may be between 1.1 mm and 1.5 mm.

In some non-limiting embodiments or aspects, the distal portion may defines a second diameter and the proximal portion may define a third diameter, wherein the second diameter may be less than the third diameter.

In some non-limiting embodiments or aspects, the intermediate portion may define a fourth diameter at least 1% smaller than the second diameter and at least 0.5% smaller than the third diameter.

Further examples of the present disclosure will now be described in the following numbered clauses.

Clause 1: A stopper adapted for use within a syringe barrel, the stopper comprising: a main body defining a proximal end, a distal end, and a cylindrical sidewall extending between the proximal end and the distal end, wherein the main body defines a first diameter; at least one rib extending radially outward around a perimeter of the main body configured to form a seal with the syringe barrel; and a trim member extending radially outward around a perimeter of the main body, the trim member comprising: a distal portion defining a second diameter, a proximal portion defining a third diameter, and an intermediate portion between the distal portion and the proximal portion, the intermediate portion defining a fourth diameter at least 1% smaller than the second diameter and at least 0.5% smaller than the third diameter.

Clause 2: The stopper of clause 1, wherein the second diameter is less than or equal to 9.15 mm.

Clause 3: The stopper of clause 1, wherein the second diameter is between 8.85 mm and 9.16 mm.

Clause 4: The stopper of any of clauses 1-3, wherein the fourth diameter is between 8.85 mm and 9.08 mm.

Clause 5: The stopper of any of clauses 1-4, wherein the third diameter is between 8.85 mm and 9.12 mm.

Clause 6: The stopper of any of clauses 1-5, wherein the main body defines a first diameter between 8.85 mm and 9.05 mm.

Clause 7: The stopper of any of clauses 1-6, wherein the distal portion includes a convex segment.

Clause 8: The stopper of any of clauses 1-7, wherein at least one of the proximal portion and the intermediate portion includes a concave segment.

Clause 9: The stopper of clause 8, wherein a radius of curvature of the concave segment is greater than a radius of curvature of the convex segment.

Clause 10: The stopper of clause 8 or clause 9, wherein the second diameter is less than the third diameter.

Clause 11: The stopper of any of clauses 1-10, wherein the trim member includes a concave segment extending from the distal portion to the proximal portion.

Clause 12: The stopper of clause 11, wherein the second diameter is less than the third diameter.

Clause 13: A stopper adapted for use within a syringe barrel, the stopper comprising: a main body defining a proximal end, a distal end, and a cylindrical sidewall extending between the proximal end and the distal end, wherein the main body defines a first diameter; at least one rib extending radially outward around a perimeter of the main body configured to form a seal with the syringe barrel; and a trim member extending radially outward around a perimeter of the main body, the trim member comprising: a distal portion including a convex segment, a proximal portion, and an intermediate portion between the distal portion and the proximal portion, wherein at least one of the proximal portion and the intermediate portion includes a concave segment.

Clause 14: The stopper of clause 13, wherein a radius of curvature of the concave segment is greater than a radius of curvature of the convex segment.

Clause 15: The stopper of clause 13 or clause 14, wherein the radius of curvature of the convex segment is between 0.3 mm and 0.7 mm.

Clause 16: The stopper of any of clauses 13-15, wherein the radius of curvature of the concave segment is between 1.1 mm and 1.5 mm.

Clause 17: The stopper of any of clauses 13-16, wherein the distal portion defines a second diameter and the proximal portion defines a third diameter, wherein the second diameter is less than the third diameter.

Clause 18: The stopper of any of clauses 13-17, wherein the intermediate portion defines a fourth diameter at least 1% smaller than the second diameter and at least 0.5% smaller than the third diameter.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
Fig. 1 is a side view of a stopper adapted for attachment with a plunger rod for use within a syringe barrel in accordance with an embodiment of the present disclosure.
Fig. 2 is a top perspective view of the stopper of Fig. 1 in accordance with an embodiment of the present disclosure.
Fig. 3 is a cross-sectional side view of the stopper of Fig. 1 in accordance with an embodiment of the present disclosure.
Fig. 4 is a partial close-up view of a portion of the stopper of Fig. 1 in accordance with an embodiment of the present disclosure.
Fig. 5A is a side view of an example of a prior art stopper.
Fig. 5B is a side view of an example of the stopper of Fig. 5A, wherein the distal end of the stopper has been removed.
Fig. 5C is a side view of an example of the stopper of Fig. 5A, wherein the distal end and distal portion of a trim member of the stopper has been removed.
Fig. 5D is a side view of an example of the stopper of Fig. 5A, wherein the distal end and a trim member of the stopper has been removed.
Fig. 6 is a graph showing examples of gliding performances of the stoppers of Figs. 5A-5D in batches a and b.
Fig. 7 is a graph illustrating the empty gliding performance for stoppers having a distal portion of a trim member at a designated diameter in accordance with an embodiment of the present disclosure.
Fig. 8 is a graph illustrating the empty gliding performance for stoppers having an intermediate portion of a trim member at a designated diameter in accordance with an embodiment of the present disclosure.
Fig. 9 is a graph illustrating the empty gliding performance for stoppers having a proximal portion of a trim member at a designated diameter in accordance with an embodiment of the present disclosure.
Figs. 10A-F are side views of examples of trim member shapes in accordance with an embodiment of the present disclosure.
Fig. 11 is a graph illustrating the radial forces exerted by the trim members of Figs. 10A-10F.
Fig. 12 is a graph illustrating comparative gliding performance between the prior art stopper of Fig. 5A and the example of a stopper shown in Figs. 1-4 and described herein constructed in accordance with an embodiment of the present disclosure.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the concept as it is oriented in the drawing figures. However, it is to be understood that the concept may assume various alternative variations, except where expressly specified to the contrary. It is also to be understood that the specific devices illustrated in the attached drawings, and described in the following specification, are simply exemplary embodiments of the concept. Hence, specific dimensions and other physical characteristics related to the embodiments disclosed herein are not to be considered as limiting.

The word "comprising" and "comprises", and the like, does not exclude the presence of elements or steps other than those listed in any claim or the specification as a whole. In the present specification, "comprises" means "includes" and "comprising" means "including." The term "at least" is synonymous with "greater than or equal to." The terms "first", "second", and the like are not intended to refer to any particular order or chronology, but refer to different conditions, properties, or elements.

All numbers used in the specification and claims are to be understood as being modified in all instances by the term "about". By "about" is meant within plus or minus twenty-five percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

Also, for purposes of the description of the present disclosure, the term "distal end" is intended to refer to the end of the syringe from which the needle projects and the end of the stopper which is closer to the syringe needle, whereas the term "proximal end" is intended to refer to the end of the syringe closer to the holder of the syringe and furthest from the needle tip and the end of the stopper furthest from the needle tip.

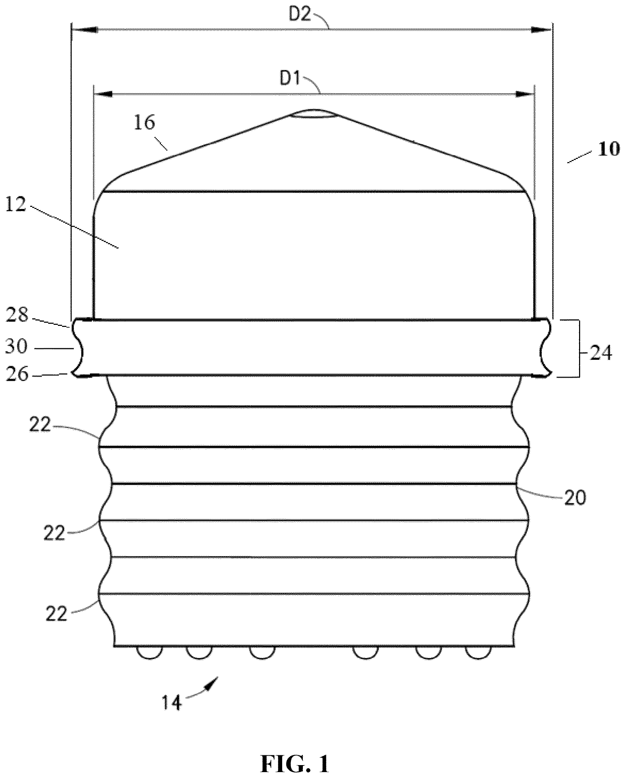

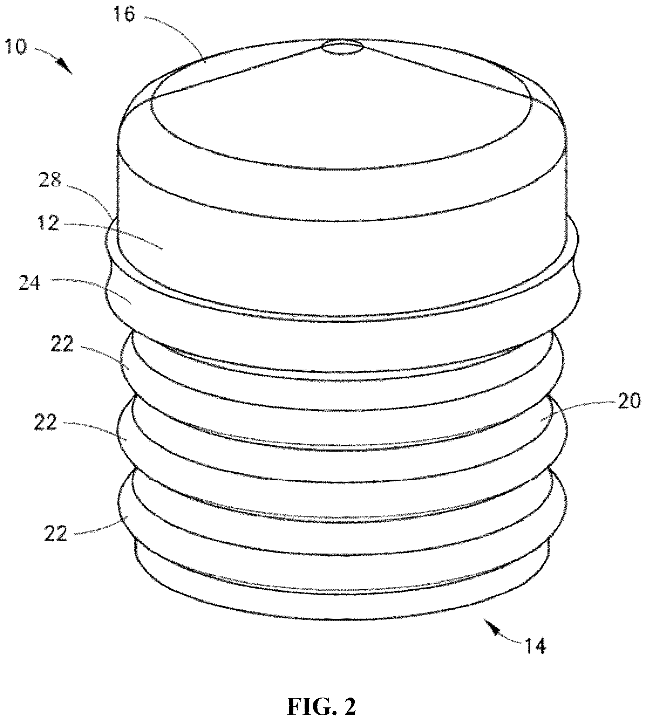

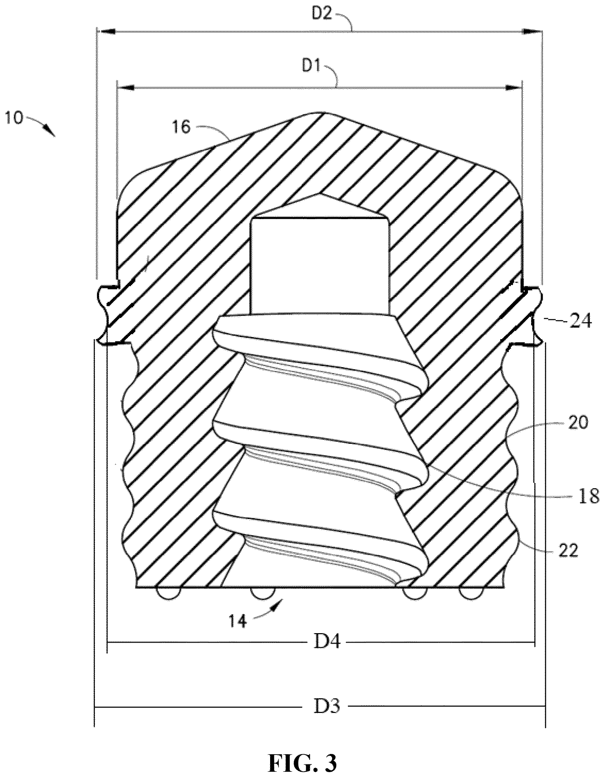

Reference is now made to Figs. 1-4 which show a stopper, generally indicated as 10, adapted for attachment with a plunger rod (not shown) for use within a syringe barrel (not shown) in accordance with an embodiment of the disclosure. The stopper 10 generally includes or defines a main body 12 with a proximal end 14, a distal end 16, and a cylindrical sidewall 20 extending between the proximal end 14 and the distal end 16. The proximal end 14 may be open or otherwise define a feature 18, such as threading or other engageable mechanism, adapted to receive an attachment portion (not shown) of the plunger rod or other actuating, dispensing mechanism. The distal end 16 may be conically shaped or otherwise configured to interact with and dispense a substance from a syringe barrel. A barrier film may be positioned onto or adjacent to the distal end 16 to provide a barrier between the contents of the syringe and the stopper material. The material used for a barrier film may include ethylene tetrafluoroethylene (ETFE), however, it can be appreciated that other known types of materials exhibiting barrier properties can be used.

The stopper 10 may further include at least one rib 22, in some instances two or three ribs, extending radially outward around a perimeter of the main body 12. The at least one rib 22 may be configured to ensure the seal of the stopper 10 with the barrel or container in which the stopper 10 is positioned. The presence of several ribs ensures the container closure integrity. The stopper 10 may include or define first diameter D1. The diameter D1 may be defined or measured across a distal portion of the main body 12 of the stopper 10.

The stopper 10 further includes a trim member 24 extending radially outward around a perimeter of the main body 12. The trim member 24 generally includes or defines a protruding feature where individual stoppers 10 are trimmed from a molding sheet (not shown) during a trimming step following manufacturing. According to one embodiment, the stopper 10 may be molded in two parts and the trim member 24 may be located between the distal end 16 and the proximal end 14. The trim member 24 may be longitudinally positioned between the distal end 16 and a distal-most rib of the at least one rib 22. The trim member 24 may include or define a proximal portion 26, a distal portion 28, and an intermediate portion 30 positioned or defined between the proximal and distal portions 26, 28. The distal portion 28 may include or define a second diameter D2, the proximal portion 26 may include or define a third diameter D3, and the intermediate portion 30 may include or define a fourth diameter D4.

The trim member 24 may include or define the largest diameter of the stopper 10 and thus may be in contact with the inner surface of the syringe barrel. The diameter of the trim member 24 may thus be larger than the diameter of the inner surface of the syringe barrel. In accordance with the present disclosure, the trim member 24 may be formed by controlling the geometry/shape, size, and/or diameter with respect to the diameter of the stopper 10 itself in order to reduce gliding forces of the stopper 10 within the barrel. It can be appreciated that stoppers and barrels can have different diameters depending upon the size of the syringe and/or injection device, as long as at least a portion of the stopper 10, such as the at least one rib 22 and/or the trim member 24, are capable of forming an active seal with respect to the inner surface of the barrel so as to prevent liquid from seeping or escaping past the rearward or proximal end 14 of the stopper 10, thus ensuring the container closure integrity.

As discussed herein, the present disclosure is directed to an optimized stopper design through dimensional and geometry/shape control to produce a stopper 10 having lower gliding forces and to avoid the adverse impact of stress concentration typically generated by trimming processes. The gliding force (either for filled or empty syringes) is directly related to a primary product parameter of the syringe, i.e. the injection time of the drug contained in the syringe. This key parameter is especially important for syringes used in auto-injectors, as these devices must comply with a defined injection time. However, existing stopper designs have trim members that demonstrate high variability regarding their gliding performances, which can negatively and unpredictably affect a resulting injection time. Moreover, the trim member 24 and portions thereof provide a significant contribution to the gliding performance of the stopper 10 compared to the ribs or other portions of the stopper 10.

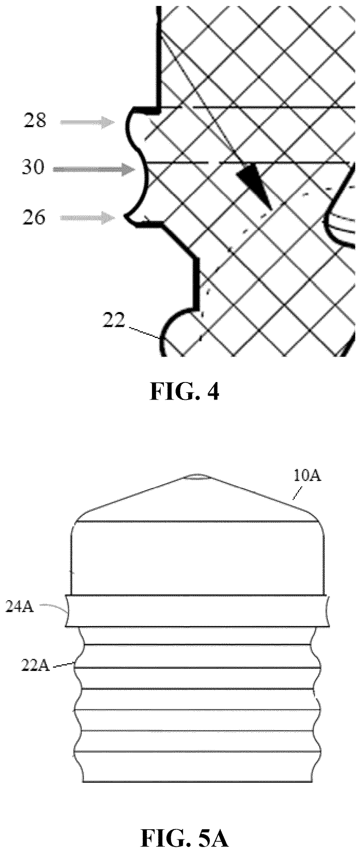

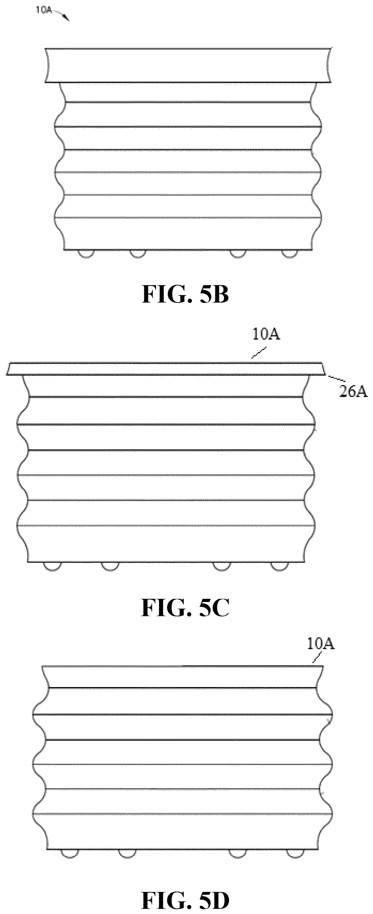

In the present disclosure, testing has been done on two different prior art stopper batches, batches "a" and "b", to demonstrate that the trim member 24 provides a significant contribution to the gliding performance of the stopper 10. Fig. 5A shows a stopper 10A comprised of an entire stopper body including a distal end 16A, a trim member 24A and a plurality of ribs 22A. Fig. 5B shows the stopper 10A comprised of both the trim member 24A and the ribs 22A, but wherein the distal end 16A has been removed. Fig. 5C shows the stopper 10A comprised of just a proximal portion 26A of the trim member 24A and the ribs 22A, but wherein the distal end 16A has been removed. Fig. 5D shows the stopper 10A comprised of only the ribs 22A, wherein the distal end 16A and trim member 24A have been removed. The stoppers tested and analyzed had a main body diameter D1 of 8.95 mm with a ± 0.10 mm variance or tolerance.

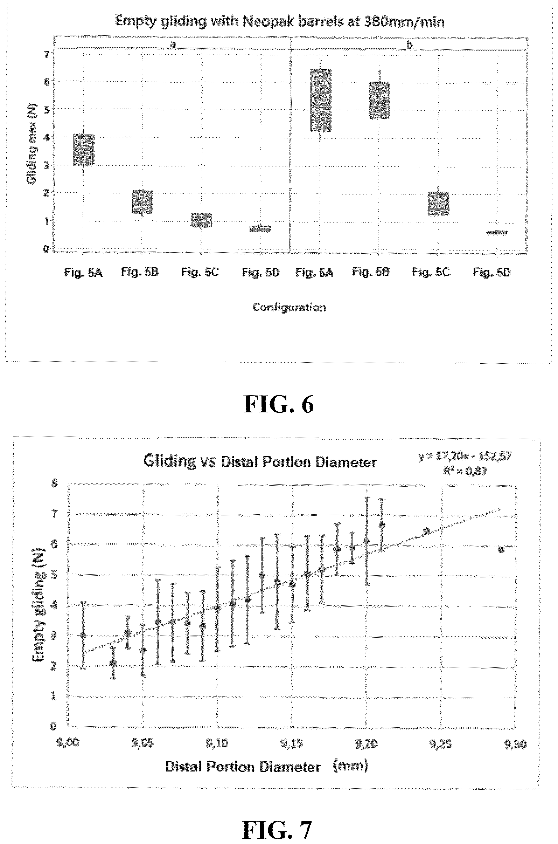

Fig. 6 shows a graph comparing gliding forces of batches "a" and "b" of each of the stopper variations shown in Figs. 5A-5D. The batches present a range of gliding performances for a given design due to potential manufacturing or operating variances with the stoppers, syringes, etc. As shown in the graph, the stoppers with the features shown in Figs. 5A-5B have substantially higher gliding forces compared to the stoppers with the features shown in Figs. 5C-5D, which demonstrates that the trim member 24, and, in particular, the distal portion 28 of the trim member 24, is the main contributing factor in the differences in gliding performances. Accordingly, the present disclosure has identified a relationship between the distal portion 28 of the trim member 24 and its functional performance (gliding).

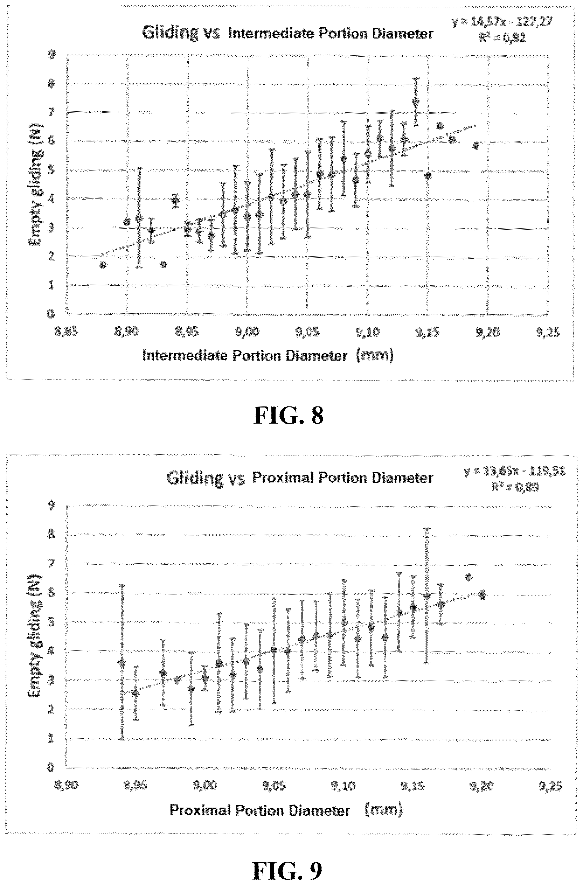

Figs. 7-9 show examples of empty gliding performances (measure of the gliding force with an empty syringe) with respect to ranges of diameters for each of the proximal portion 26, distal portion 28, and intermediate portion 30 of the trim member 24. The measured parameters indicate improved, beneficial performance when the proximal portion is between approximately 8.85mm and 9.12mm, the distal portion is between approximately 8.85mm and 9.16mm, and the intermediate portion is between approximately 8.85mm and 9.08mm for a stopper having a main body diameter D1 of 8.95 mm with a ± 0.10 mm, e.g., between 8.85 mm and 9.05 mm. The analysis also indicates improved glide performance in which stopper 10 includes the distal portion 28 with a diameter D2 not exceeding 9.15 mm, the intermediate portion 30 with a diameter D4 at least 1% smaller than the diameter D2 of the distal portion 28, and the intermediate portion 30 with a diameter D4 at least 0.5% smaller than the diameter D3 of the proximal portion 26.

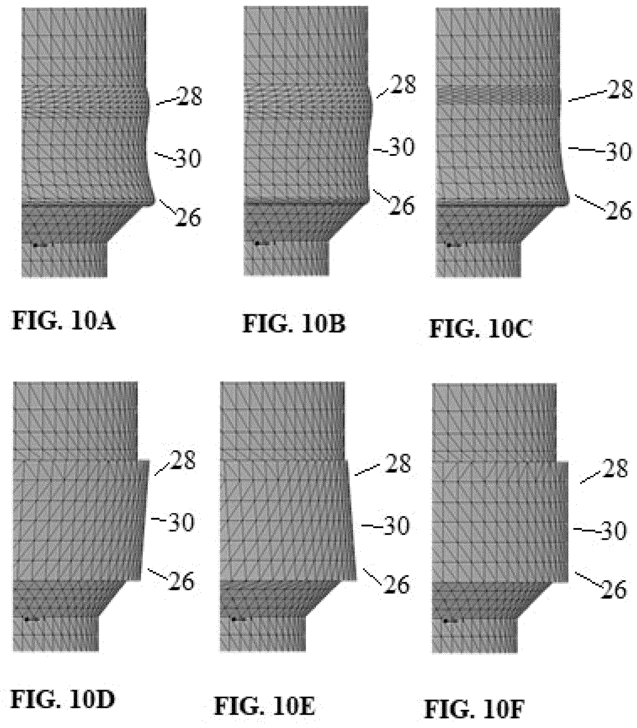

The shape of the trim member 24 also affects gliding performance. In particular, the cross-sectional geometry of the outer lateral edge or sidewall extending between the distal portion 28 and the proximal portion 26 of the trim member 24, and the resulting forces the lateral, exterior edge experiences when interacting with a syringe barrel, affects gliding performance and the resulting injection time, as described herein. Referring now to Figs. 10A-10F, examples of the stopper 10 with various profile geometries of the trim member 24 are shown.

The example of the stopper 10 shown in Fig. 10A includes a curvilinear shape profile for the trim member 24, which is the same shape profile of the trim member 24 illustrated in Figs. 1-4. In this example, the trim member 24 includes or defines an outwardly extending convex segment at or about the distal portion 28, which extends into a concave segment at or about the intermediate portion 30, with the proximal portion 26 extending outwards with a larger diameter than the center or innermost region of the concave segment. The concave segment may extend along at least a portion of both the proximal portion 26 and the intermediate portion 30. In other words, the distal portion 28 may include the convex segment, while the intermediate portion 30 and proximal portion 26 may collectively include the concave segment.

In one non-limiting example, the convex segment may include or define a radius of curvature of approximately 0.5 mm. In another example, the convex segment may include or define a radius of curvature between 0.3 mm and 0.7 mm. In one non-limiting example, the concave segment may include or define a radius of curvature of approximately 1.30 mm. In another example, the concave segment may include or define a radius of curvature between 1.10 mm and 1.50 mm. The radius of curvature of the concave segment may be greater than the radius of curvature of the convex segment.

The example of the stopper 10 shown in Fig. 10B includes another example of a curvilinear shape profile for the trim member 24. In this example, the trim member 24 includes or defines an outwardly extending convex segment at or about the distal portion 28, which extends into a concave segment at or about the intermediate portion 30, with the proximal portion 26 extending outwards with a larger diameter than the center or innermost region of the concave segment. The concave segment may extend along at least a portion of both the proximal portion 26 and the intermediate portion 30. In other words, the distal portion 28 may include the convex segment, while the intermediate portion 30 and proximal portion 26 may collectively include the concave segment. This example of the stopper 10 has a concave segment with a less pronounced curvature compared to the concave segment of the stopper 10 of Fig. 10A.

In one non-limiting example, the convex segment may include or define a radius of curvature of approximately 0.5 mm. In another example, the convex segment may include or define a radius of curvature between 0.3 mm and 0.7 mm. In one non-limiting example, the concave segment may include or define a radius of curvature of approximately 2.26 mm. In another example, the concave segment may include or define a radius of curvature between 2.0 mm and 2.50 mm. The radius of curvature of the concave segment may be greater than the radius of curvature of the convex segment.

The example of the stopper 10 shown in Fig. 10C includes another example of a curvilinear shape profile for the trim member 24. In this example, the trim member 24 includes or defines a generally concave segment extending through the distal portion 28, the intermediate portion 30, and the proximal portion 26 extending outwards with a larger diameter in the proximal portion 26 compared to the distal portion 28. In one non-limiting example, the concave segment may include or define a radius of curvature of approximately 2.26 mm. In another example, the concave segment may include or define a radius of curvature between 2.0 mm and 2.50 mm.

The examples of the stoppers 10 shown in Figs. 10D-10F each include a substantially linear side profile for the respective outer edges or sidewalls of the trim member 24. For example, the example of the stopper 10 shown in Fig. 10D includes a trim member 24 with a substantially linear profile extending from the distal portion 28 to the proximal portion 26, where the distal portion 28 has a larger diameter than the proximal portion 26, resulting in the substantially linear sidewall of the trim member 24 angling inward towards the main body of the stopper as it traverses from the distal portion 28 to the proximal portion 26.

The example of the stopper 10 shown in Fig. 10E also includes a trim member 24 with a substantially linear profile extending from the distal portion 28 to the proximal portion 26, but at an inverted angular orientation compared to the stopper 10 of Fig. 10D. In particular, for the stopper 10 of Fig. 10E, the distal portion 28 has a smaller diameter than the proximal portion 26, resulting in the substantially linear sidewall of the trim member 24 angling outwards as it traverses from the distal portion 28 to the proximal portion 26.

The example of the stopper 10 shown in Fig. 10F also includes a trim member 24 with a substantially linear profile extending from the distal portion 28 to the proximal portion 26, but at a substantially vertical orientation or otherwise in alignment with a longitudinal axis of the stopper 10. In particular, for the stopper 10 of Fig. 10F, the distal portion 28 has a diameter substantially equal to or the same as the dimeter of the proximal portion 26.

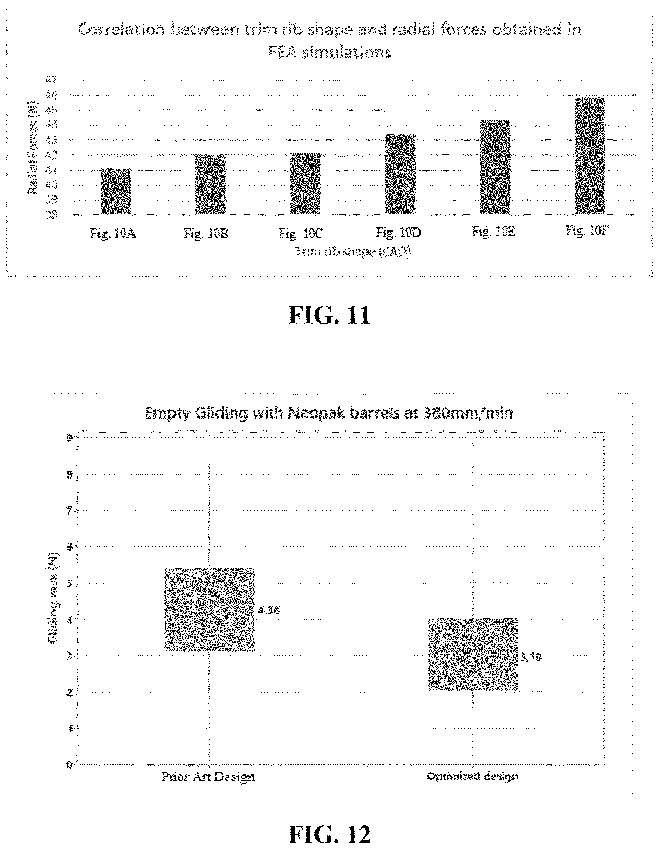

Now referring to Fig. 11, a graph is shown illustrating the radial forces exerted or experienced by the trim members 24 having the shapes and profiles shown in Figs. 10A-10F and described herein. As shown in the graph, the curvilinear trim members of the stoppers in Figs. 10A-10C have substantially better performance with lower radial forces compared to the substantially linear profile shapes of the stoppers in Figs. 10D-10F.

As shown by the analysis and description herein, existing stoppers may be improved by incorporating specific parameters for the diameters of proximal, intermediate, and distal portions of a trim member. Such diameters and other dimensional optimization can improve a stopper's glide performance and radial force exertion, even if the stopper has a trim member shape profile that is less than optimal. Moreover, existing stoppers may be improved by incorporating specific shape profiles for proximal, intermediate, and distal portions of a trim member. Such shape profile optimization can improve a stopper's glide performance and radial force exertion, even if the stopper has diameter or dimensional characteristics that are less than optimal. Finally, a stopper implementing both specific parameters for the diameters of proximal, intermediate, and distal portions of a trim member and specific shape profiles for proximal, intermediate, and distal portions of the trim member may provide significantly improved performance. For example, Fig. 12 is a graph comparing the gliding forces and performance of the prior art stopper shown in Fig. 5A with the stopper 10 as shown in Figs. 1-4 and described herein. The graph shows that with the described optimization, a minimum of 30% reduction in maximum gliding force can be achieved.

In order to control the shape and/or geometry, and/or diameter of the trim member 24, the trim member 24 may be formed with a cutting tool, as opposed to a molding process to form the stopper main body. The cutting tool can be a pre-stretched cutting die. Alternatively, the cutting tool can be a progressive cutting-edge die, which is a cutting tool that uses a wavy cutting edge engaging progressively on the rubber instead of a one-cut stroke die, which is prone to rubber displacement due to compressive stresses and the Poisson coupling effect. It can be appreciated that other types of cutting tools can be used to separate the stopper from the molding sheet.

In accordance with another aspect, the present disclosure is directed to a method for forming consistent gliding forces for a series of stoppers 10 adapted for attachment with a series of plunger rods for use in a series of syringe barrels. The method comprises providing a series of stoppers 10, each of the stoppers 10 comprising a main body 12, defining a rearward or proximal end 14, a front or distal end 16, and a cylindrical sidewall 20 extending between the rearward end 14 and the front end 16. The proximal end 14 may be adapted to receive a front distal end attachment portion (not shown) of the plunger rod. At least one rib 22 may be provided that extends radially outward around a perimeter of the main body 12. The at least one rib 22 may comprise three ribs, and may be configured for forming an active seal with the syringe barrel. A trim member 24 extends radially outward around a perimeter of main body 12.

The trim member 24 includes a proximal portion 26, a distal portion 28, and an intermediate portion 30 disposed or otherwise extending between the proximal and distal portions 26, 28. The stopper 10 may have one or more relative diameters of the portions thereof configured for reducing gliding forces of the stopper 10 within the barrel as disclosed herein, and/or one or more shape profiles as disclosed herein. The dimensions and shape characteristics of the various portions of each trim member 24 of each of the series of stoppers 10 may be controlled by using a cutting tool. The cutting tool can be one of a pre-stretched cutting die or a progressive cutting-edge die. Other known cutting dies can be used for cutting the trim member 24 wherein the shape, geometry, and/or diameter of the trim member 24 is controlled. The use of a cutting tool enables closer control and tolerances of the trim rib dimensions and shape, resulting in a controlled and consistent trim member 24. The method can further include applying a barrier film on and/or adjacent to the distal end 16 of each of the stoppers 10, such that the barrier film extends to the trim member 24. The method may further comprise removing the excess barrier material from the stoppers 10.

While the disclosure has been described as having exemplary designs, the present disclosure can be further modified within the spirit and scope of this disclosure. This application is, therefore, intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A stopper adapted for use within a syringe barrel, the stopper comprising:
a main body defining a proximal end, a distal end, and a cylindrical sidewall extending between the proximal end and the distal end, wherein the main body defines a first diameter;
at least one rib extending radially outward around a perimeter of the main body configured to form a seal with the syringe barrel; and
a trim member extending radially outward around a perimeter of the main body, the trim member comprising:
a distal portion defining a second diameter,
a proximal portion defining a third diameter, and
an intermediate portion between the distal portion and the proximal portion, the intermediate portion defining a fourth diameter at least 1% smaller than the second diameter and at least 0.5% smaller than the third diameter.

2. The stopper of claim 1, wherein the second diameter is less than or equal to 9.15 mm.

3. The stopper of claim 1, wherein the second diameter is between 8.85 mm and 9.16 mm.

4. The stopper of claim 1, wherein the fourth diameter is between 8.85 mm and 9.08 mm.

5. The stopper of claim 1, wherein the third diameter is between 8.85 mm and 9.12 mm.

6. The stopper of claim 1, wherein the main body defines a first diameter between 8.85 mm and 9.05 mm.

7. The stopper of claim 1, wherein the distal portion includes a convex segment.

8. The stopper of claim 7, wherein at least one of the proximal portion and the intermediate portion includes a concave segment.

9. The stopper of claim 8, wherein a radius of curvature of the concave segment is greater than a radius of curvature of the convex segment.

10. The stopper of claim 8, wherein the second diameter is less than the third diameter.

11. The stopper of claim 1, wherein the trim member includes a concave segment extending from the distal portion to the proximal portion.

12. The stopper of claim 11, wherein the second diameter is less than the third diameter.

13. A stopper adapted for use within a syringe barrel, the stopper comprising:
a main body defining a proximal end, a distal end, and a cylindrical sidewall extending between the proximal end and the distal end, wherein the main body defines a first diameter;
at least one rib extending radially outward around a perimeter of the main body configured to form a seal with the syringe barrel; and
a trim member extending radially outward around a perimeter of the main body, the trim member comprising:
a distal portion including a convex segment,
a proximal portion, and
an intermediate portion between the distal portion and the proximal portion, wherein at least one of the proximal portion and the intermediate portion includes a concave segment.

14. The stopper of claim 13, wherein a radius of curvature of the concave segment is greater than a radius of curvature of the convex segment;
wherein, optionally, the radius of curvature of the convex segment is between 0.3 mm and 0.7 mm; and
wherein, optionally, the radius of curvature of the concave segment is between 1.1 mm and 1.5 mm.

15. The stopper of claim 13, wherein the distal portion defines a second diameter and the proximal portion defines a third diameter, wherein the second diameter is less than the third diameter; and
wherein, optionally, the intermediate portion defines a fourth diameter at least 1% smaller than the second diameter and at least 0.5% smaller than the third diameter.
